# EUROPEAN PATENT APPLICATION

(11) **EP 1 977 675 A1**
(43) Date of publication of application: **08.10.2008**
(21) Application number: 06810321.7
(22) Date of filing: 20.09.2006
(51) Int. Cl.: A61B 1/00

(54) **METHOD OF MANUFACTURING ENDOSCOPE INSERTION PART**

(30) Priority: 23.01.2006 JP 2006013864
(71) Applicant: Olympus Corporation, Tokyo 151-0072 (JP)
(72) Inventor: ITO, Yoshiaki, . (JP)
(74) Representative: Winter, Brandl, Fürniss, Hübner Röss, Kaiser, Polte Partnerschaft Patent- und Rechtsanwaltskanzlei
(86) International application number: PCT/JP2006/318625
(87) International publication number: WO 2007/083413

(57) **Abstract**

The present invention provides a simple method of manufacturing an insertion portion of an endoscope.

The method includes preparing a first bending part (36) which includes a first tubular portion (38) which is elastic and is substantially tube-shaped, and a discontinuous portion which extends across the first tubular portion (38) which extends in a peripheral direction, preparing a second bending part (36) which includes a second tubular portion (38) which is substantially tube-shaped, changing a diameter of the first tubular portion (38) by deforming the first tubular portion (38) elastically, aligning a projecting portion (42) provided with one of the first and second bending parts (36) and extending in a radial direction of the tubular portion (38) with a receiving portion (46) provided with the other of the first and second bending parts (36) by moving the first and second bending parts (36) relatively, and connecting the first and second bending parts (36) swingably relative to each other by releasing the elastic deformation of the first tubular portion (38) to return the diameter thereof to its original state and inserting the projecting portion (42) into the receiving portion (46) rotatably.

## Description

### Technical Field

The present invention relates to a method of manufacturing an insertion portion of an endoscope, the insertion portion including a bending portion to be bent.

### Background Art

An insertion portion of an endoscope is provided with a bending portion to be bent. In a bending tube serving as a frame for the bending portion, a plurality of cylindrical-shaped bending parts is arranged side by side concentrically. The adjacent bending parts are swingably coupled through a pair of connecting portions at symmetrical positions with respect to the central axis.

Jpn. Pat. Appln. KOKAI Publication No. 11-19032 discloses an example of the connecting portions. Tongue piece portions extend from each of end faces of each bending part. The tongue piece portions of the adjacent bending parts are overlapped with each other and are rotatably riveted.

Jpn. Pat. Appln. KOKAI Publication No. 2001-104239 discloses another example of the connecting portions. Tongue piece portions extend from each of end faces of each bending part. The tongue piece portions of the adjacent bending parts are overlapped with each other and a projecting part of the tongue piece portions of one bending part is rotatably inserted into a though hole of the tongue piece portions of the other bending part.

### Disclosure of Invention

A method of manufacturing a bending tube disclosed in Jpn. Pat. Appln. KOKAI Publication No. 11-19032 requires a series of steps of forming a plurality of continuous cylindrical bending parts by press processing, positioning the bending parts with each other such that the bending parts are arranged concentrically and through holes of tongue piece portions of the adjacent bending parts match each other, inserting a rivet into the set of matching through holes and caulking the rivet. If the tongue piece portions are tightly riveted, the tongue piece portions are difficult to rotate, leading to deterioration in operability during bending the bending portion. In post-processing, therefore, it is necessary to forcedly rotate the connected bending parts in order to provide play between the rivet and the associated tongue piece portions so that the tongue piece portions are smoothly rotated. As described above, the method of manufacturing the bending tube is complicated.

In a method of manufacturing a bending tube disclosed in Jpn. Pat. Appln. KOKAI Publication No. 2001-104239, a plurality of continuous cylindrical bending parts are formed by press processing, the bending parts are aligned with each other such that the bending parts are arranged concentrically and tongue piece portions of the adjacent bending parts are overlapped with each other. The outer tongue piece portion is pressed from the outside, thus partially projecting the tongue piece portion inwardly in the radial direction and inserting the projecting portion into the through hole of the inner tongue piece portion. As described above, the method of manufacturing the bending tube is complicated.

The present invention is made in consideration of the above problems and an object of the present invention is to provide a simple method of manufacturing an insertion portion of an endoscope.

According to an aspect of the present invention, a method of manufacturing an insertion portion of an endoscope is characterized by comprising: preparing a first bending part which includes a first tubular portion which is elastic and is substantially tube-shaped, and a discontinuous portion which extends across the first tubular portion which extends in a peripheral direction; preparing a second bending part which includes a second tubular portion which is substantially tube-shaped; changing a diameter of the first tubular portion by deforming the first tubular portion elastically; aligning a projecting portion provided with one of the first and second bending parts and extending in a radial direction of the tubular portion with a receiving portion provided with the other of the first and second bending parts by moving the first and second bending parts relatively; and connecting the first and second bending parts swingably relative to each other by releasing the elastic deformation of the first tubular portion to return the diameter thereof to its original state and inserting the projecting portion into the receiving portion rotatably.

According to a preferred aspect of the present invention, the method of manufacturing an insertion portion of an endoscope is **characterized in that** the first bending part has the projecting portion which projects outwardly in a radial direction of the first tubular portion, and changing the diameter of the first tubular portion includes reducing the diameter of the first tubular portion such that the projecting portion is moved inwardly in the radial direction of the first tubular portion.

According to a preferred aspect of the present invention, the method of manufacturing an insertion portion of an endoscope is **characterized in that** the first bending part has the projecting portion which projects inwardly in a radial direction of the first tubular portion, and changing the diameter of the first tubular portion includes increasing the diameter of the first tubular portion such that the projecting portion is moved outwardly in the radial direction of the first tubular portion.

According to a preferred aspect of the present invention, the method of manufacturing an insertion portion of an endoscope is **characterized in that** the first bending part has the receiving portion, the second bending part has the projecting portion which projects outwardly in a radial direction of the second tubular portion, and changing the diameter of the first tubular portion includes increasing the diameter of the first tubular portion such that the receiving portion is moved outwardly in a radial direction of the first tubular portion.

According to a preferred aspect of the present invention, the method of manufacturing an insertion portion of an endoscope is **characterized in that** the first bending part has the receiving portion, the second bending part has the projecting portion which projects inwardly in a radial direction of the second tubular portion, and changing the diameter of the first tubular portion includes reducing the diameter of the first tubular portion such that the receiving portion is moved inwardly in a radial direction of the first tubular portion.

According to a preferred aspect of the present invention, the method of manufacturing an insertion portion of an endoscope is characterized by further comprising: repeating all of the steps, wherein the first bending part connected to the second bending part in the connecting serves as the second bending part in the next repeated steps.

According to a preferred aspect of the present invention, the method of manufacturing an insertion portion of an endoscope is **characterized in that** the preparing the first bending part includes: forming the projecting portion and the receiving portion in a plate material, forming a first bending part preparation portion including the projecting portion, the receiving portion and a first tubular portion preparation portion for forming the first tubular portion, which is substantially plate-shaped; and forming the first tubular portion and the discontinuous portion by bending the first tubular portion preparation portion.

According to a preferred aspect of the present invention, the method of manufacturing an insertion portion of an endoscope is **characterized in that** the preparing the first bending part includes preparing the first bending part in a first line and preparing the first bending part in a second line, and the aligning and the connecting includes moving the second bending part between the first and second lines and positioning and connecting the second bending part to the first bending part in the first line or the first bending part in the second line.

According to a preferred aspect of the present invention, the method of manufacturing an insertion portion of an endoscope is **characterized in that** the forming the first tubular portion includes forming the first tubular portion with an arbitrary diameter by bending the first tubular portion preparation portion through a press processing.

According to a preferred aspect of the present invention, the method of manufacturing an insertion portion of an endoscope is characterized by further comprising joining the discontinuous portion in the first bending part after the connecting the first and second bending parts.

In the present invention, an insertion portion of an endoscope can be easily manufactured.

### Brief Description of Drawings

FIG. 1 is a perspective view illustrating an endoscope according to a first embodiment of the present invention.
FIG. 2A is a perspective view showing a bending part of a bending tube included in the endoscope according to the first embodiment of the present invention.
FIG. 2B is a perspective view showing the bending tube in the endoscope according to the first embodiment of the present invention.
FIG. 3A is a perspective view illustrating a first chuck unit of an apparatus for manufacturing an insertion portion of the endoscope according to the first embodiment of the present invention, as viewed from upstream of a forming assembly line.
FIG. 3B is a perspective view showing the first chuck unit of the apparatus for manufacturing the insertion portion of the endoscope according to the first embodiment of the present invention, as viewed from downstream of the forming assembly line.
FIG. 4A is a perspective view illustrating a second chuck unit of the apparatus for manufacturing the insertion portion of the endoscope according to the first embodiment of the present invention, as viewed from upstream of the forming assembly line.
FIG. 4B is a perspective view showing the second chuck unit of the apparatus for manufacturing the insertion portion of the endoscope according to the first embodiment of the present invention, as viewed from downstream of the forming assembly line.
FIG. 5A is a perspective view illustrating the apparatus for manufacturing the insertion portion of the endoscope according to the first embodiment of the present invention, as viewed from upstream of the forming assembly line.
FIG. 5B is a perspective view showing the apparatus for manufacturing the insertion portion of the endoscope according to the first embodiment of the present invention, as viewed from downstream of the forming assembly line.
FIG. 5C is a side view illustrating the apparatus for manufacturing the insertion portion of the endoscope according to the first embodiment of the present invention.
FIG. 5D is a top view showing the apparatus for manufacturing the insertion portion of the endoscope according to the first embodiment of the present invention.
FIG. 6A is a sectional side elevation showing a step of aligning a first bending part held by holding reducing-diameter chucks with a second bending part held by holding chucks with respect to the peripheral direction in a process of connecting the bending parts in a method of manufacturing the insertion portion of the endoscope according to the first embodiment of the present invention.
FIG. 6B is a sectional side elevation showing a step of reducing the diameter of the first bending part, which is held by the holding reducing-diameter chucks, in the process of connecting the bending parts in the method of manufacturing the insertion portion of the endoscope according to the first embodiment of the present invention.
FIG. 6C is a sectional side elevation showing a step of aligning the first bending part held by the holding reducing-diameter chucks with the second bending part held by the holding chucks with respect to the central axial direction in the process of connecting the bending parts in the method of manufacturing the insertion portion of the endoscope according to the first embodiment of the present invention.
FIG. 6D is a sectional side elevation showing a step of returning the first bending part held by the holding reducing-diameter chucks to its original state in the process of connecting the bending parts in the method of manufacturing the insertion portion of the endoscope according to the first embodiment of the present invention.
FIG. 6E is a sectional side elevation showing a step of holding the first bending part by conveying chucks in the process of connecting the bending parts in the method of manufacturing the insertion portion of the endoscope according to the first embodiment of the present invention.
FIG. 6F is a sectional side elevation showing a step of releasing the first bending part held by the holding reducing-diameter chucks and the second bending part held by the holding chucks in the process of connecting the bending parts in the method of manufacturing the insertion portion of the endoscope according to the first embodiment of the present invention.
FIG. 6G is a sectional side elevation showing a step of conveying the first bending part by the conveying chucks in the process of connecting the bending parts in the method of manufacturing the insertion portion of the endoscope according to the first embodiment of the present invention.
FIG. 6H is a sectional side elevation showing a step of holding the first bending part as the second bending part through the holding chucks in the process of connecting the bending parts in the method of manufacturing the insertion portion of the endoscope according to the first embodiment of the present invention.
FIG. 7 is a longitudinal sectional view for explaining a method of manufacturing an insertion portion of an endoscope according to a second embodiment of the present invention.
FIG. 8 is a longitudinal sectional view showing a bending tube of the insertion portion of the endoscope according to the second embodiment of the present invention.
FIG. 9 is a longitudinal sectional view showing a bending tube of an insertion portion of an endoscope according to a third embodiment of the present invention.
FIG. 10A is a front view illustrating a first chuck unit of an apparatus for manufacturing the insertion portion of the endoscope according to the third embodiment of the present invention.
FIG. 10B is a top view showing a holding increasing-diameter unit of a first chuck unit of the apparatus for manufacturing the insertion portion of the endoscope according to the third embodiment of the present invention.
FIG. 10C is a front view showing the first chuck unit for explaining a method of manufacturing the insertion portion of the endoscope according to the third embodiment of the present invention.
FIG. 10D is a top view showing the holding increasing-diameter unit of the first chuck unit for explaining the method of manufacturing the insertion portion of the endoscope according to the third embodiment of the present invention.
FIG. 11A is a perspective view illustrating a first left and right chuck unit of an apparatus for manufacturing an insertion portion of an endoscope according to a fourth embodiment of the present invention, as viewed from upstream of a forming assembly line.
FIG. 11B is a perspective view showing the first left and right chuck unit of the apparatus for manufacturing the insertion portion of the endoscope according to the fourth embodiment of the present invention, as viewed from downstream of the forming assembly line.
FIG. 12A is a perspective view illustrating a second chuck unit of the apparatus for manufacturing the insertion portion of the endoscope according to the fourth embodiment of the present invention, as viewed from upstream of the forming assembly line.
FIG. 12B is a perspective view showing the second chuck unit of the apparatus for manufacturing the insertion portion of the endoscope according to the fourth embodiment of the present invention, as viewed from downstream of the forming assembly line.
FIG. 13A is a perspective view showing the apparatus for manufacturing the insertion portion of the endoscope according to the fourth embodiment of the present invention, as viewed from upstream of the forming assembly line.
FIG. 13B is a perspective view showing the apparatus for manufacturing the insertion portion of the endoscope according to the fourth embodiment of the present invention, as viewed from downstream of the forming assembly line.
FIG. 13C is a side view showing the apparatus for manufacturing the insertion portion of the endoscope according to the fourth embodiment of the present invention.
FIG. 13D is a top view showing the apparatus for manufacturing the insertion portion of the endoscope according to the fourth embodiment of the present invention.
FIG. 14 is a sectional side elevation showing a bending tube of an endoscope according to a fifth embodiment of the present invention.
FIG. 15A includes front views each showing upper and lower press units of an apparatus for manufacturing an insertion portion of the endoscope according to the fifth embodiment of the present invention.
FIG. 15B is a longitudinal sectional view for explaining a method of manufacturing the insertion portion of the endoscope according to the fifth embodiment of the present invention.
FIG. 16A is a longitudinal sectional view showing a bending tube of an insertion portion of an endoscope according to a reference embodiment of the present invention.
FIG. 16B is a side view showing the bending tube of the insertion portion of the endoscope according to the reference embodiment of the present invention.

### Best Mode for Carrying Out the Invention

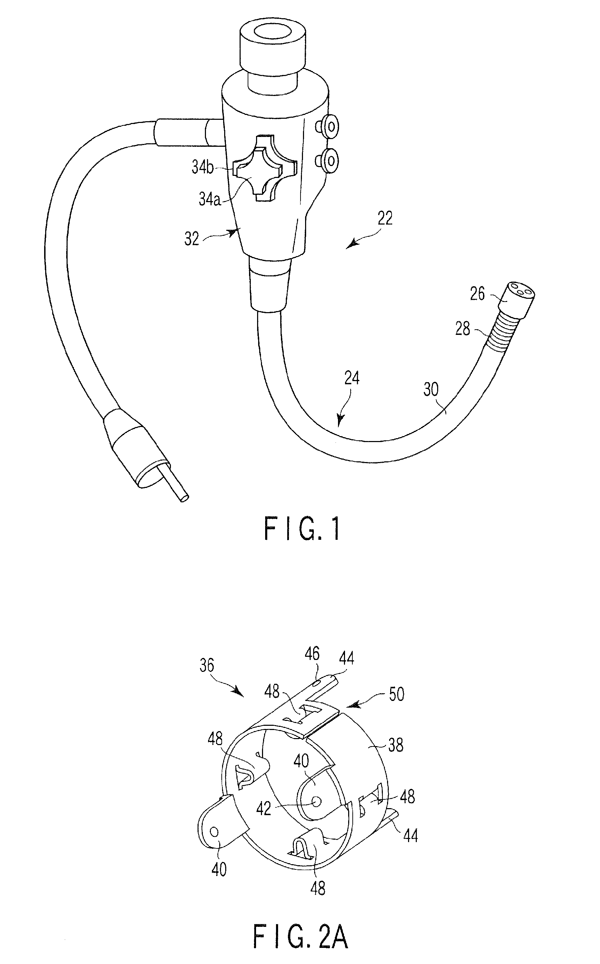

A first embodiment of the present invention will be described below with reference to FIGS. 1 to 6H.

Referring to FIG. 1, an endoscope 22 according to the present embodiment includes an elongated insertion portion 24 to be inserted into a body cavity. The insertion portion 24 includes a distal end portion 26, a bending portion 28 to be bent, and an elongated flexible insertion tube 30 connected in this order from the distal end thereof. The proximal end of the insertion portion 24 is connected to an operation portion 32 which is held and operated by an operator. The operation portion 32 includes an up-and-down bending operation knob 34a and a left-and-right bending operation knob 34b for the bending operation of the bending portion 28.

Referring to FIGS. 1 to 2B, in a bending tube 34, serving as a frame of the bending portion 28, a plurality of elastic, thin, and cylindrical bending parts 36 is arranged side by side concentrically.

A pair of projecting portion tongue piece portions 40 protrudes from one end face of a cylindrical portion 38, serving as a tubular portion, of bending part 36 in the central axial direction of the bending part 36 at symmetrical positions with respect to the central axis. In a connecting portion between the cylindrical portion 38 and the projecting portion tongue piece portion 40 of bending part 36, a step projecting inwardly in the radial direction is formed such that the height of the step is greater than the thickness of the bending part 36. The projecting portion tongue piece portion 40 is disposed substantially parallel to the cylindrical portion 38 inside the cylindrical portion 38 in the radial direction. A projecting portion 42 is provided with the outer surface of the projecting portion tongue piece portion 40. On the other hand, a pair of receiving portion tongue piece portions 44 protrudes from the other end face of the bending part 36 in the central axial direction of the bending part 36 at symmetrical positions with respect to the central axis, the positions of the receiving portion tongue piece portions 44 being obtained by rotating the pair of projecting portion tongue piece portions 40 by substantially 90° when viewed in the central axial direction of the bending part 36. The receiving portion tongue piece portions 44 are arranged substantially parallel to the cylindrical portion 38 of the bending part 36. A through hole 46, serving as a receiving portion, is formed through the receiving portion tongue piece portion 44.

The adjacent bending parts 36 are arranged such that the pair of receiving portion tongue piece portions 44 of one bending part 36 are deviated from those of the other bending part 36 by substantially 90° when viewed in the central axial direction of the bending part 36. In addition, the projecting portion tongue piece portions 40 of the one bending part 36 are overlapped with the receiving portion tongue piece portions 44 of the other bending part 36 and the projecting portion 42 of the projecting portion tongue piece portion 40 is rotatably inserted into the through hole 46 of the corresponding receiving portion tongue piece portion 44. In the adjacent bending parts 36, the projecting portions 42 are rotated in the associated through holes 46, respectively, so that both the bending parts 36 are swung relative to each other. The swinging direction of a bending part 36 preceding to a certain bending part 36 with respect to the certain bending part 36 is substantially orthogonal to that of a bending part 36 next to the certain bending part 36 with respect to the certain bending part 36. Combining the bending parts 36 swinging as described above enables the bending tube 34 to bend in any direction.

The bending part 36 has wire holders 48 through which operating wires for the bending operation of the bending portion 28 are inserted, respectively. The wire holder 48 is formed in such a manner that a portion between slits which extend side by side in peripheral direction in the cylindrical portion 38 of the bending part 36 is protruded inwardly in the radial direction of the bending part 36. In the bending part 36, the wire holders 48 are arranged at positions corresponding to upper, lower, left, and right sides of the field of view for observation of the endoscope 22. Operating wires for upward, downward, leftward, and rightward bending operations are inserted through the respective wire holders 48 at the upper, lower, left, and right positions. Thus, the bending portion 28 can be bent upward, downward, leftward, and rightward.

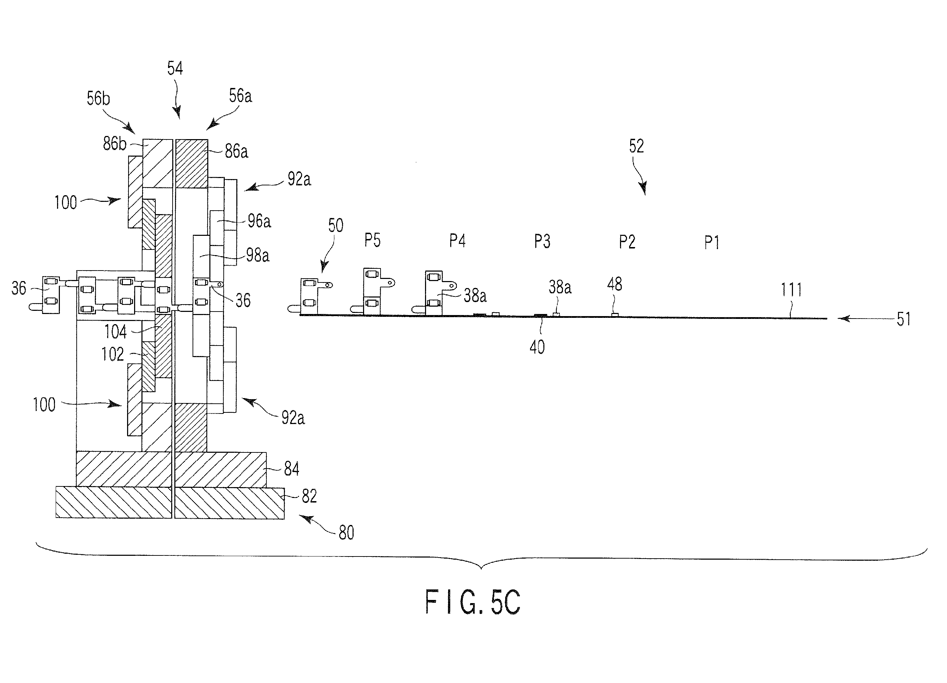

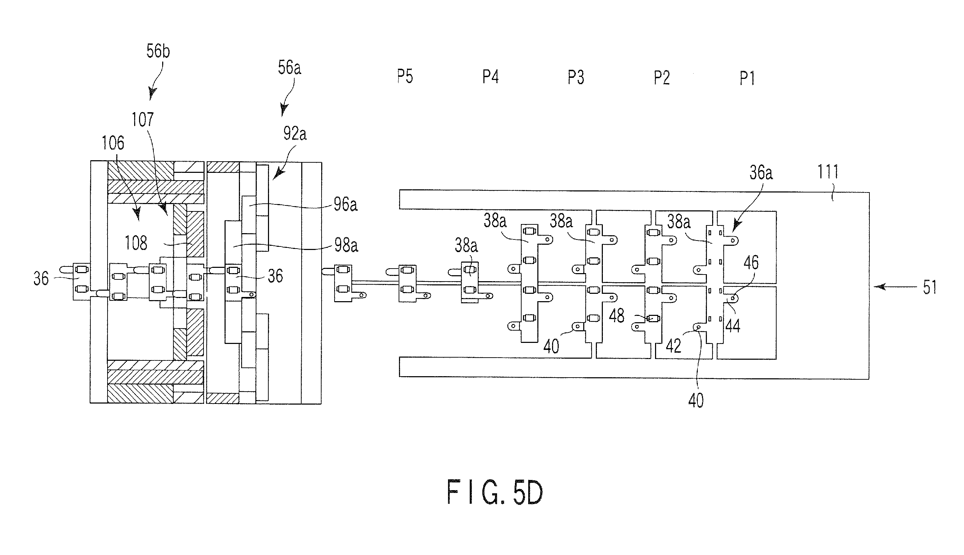

As will be described in further detail below, the cylindrical portion 38 of the bending part 36 is formed in such a manner that a plate-shaped cylindrical portion preparation potion 38a (refer to FIG. 5D) is shaped into a cylinder by press processing. Accordingly, the cylindrical portion 38 of the bending part 36 has a discontinuous portion 50 which extends in the axial direction of the bending part 36 across the cylindrical portion 38 which extends in the peripheral direction. The bending part 36 is elastically deformed to change the width of the discontinuous portion 50, thus changing the diameter of the cylindrical portion 38.

A method of manufacturing the insertion portion 24 of the endoscope according to the present embodiment will now be described.

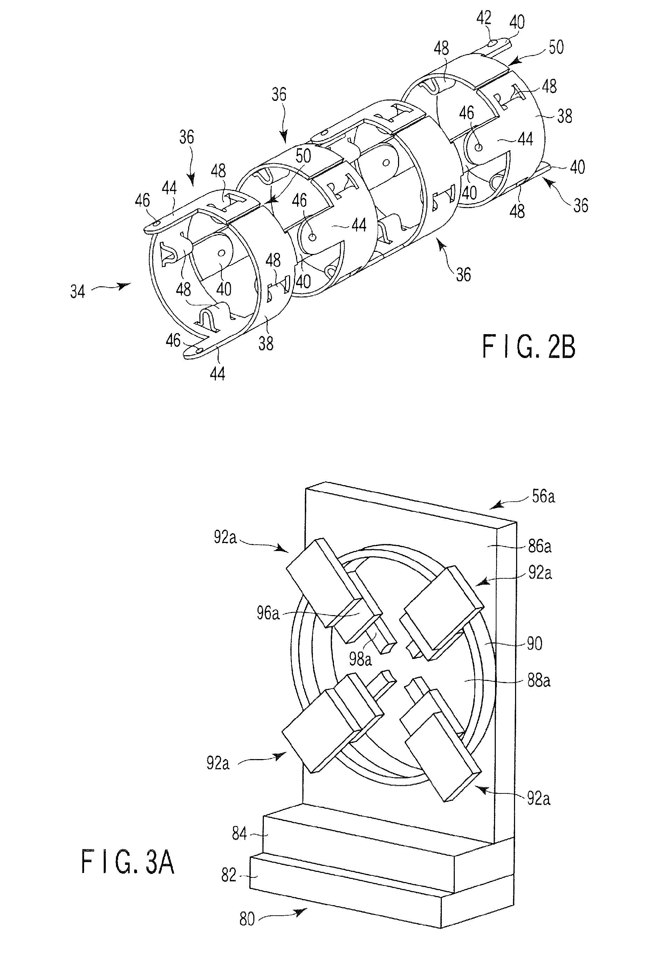

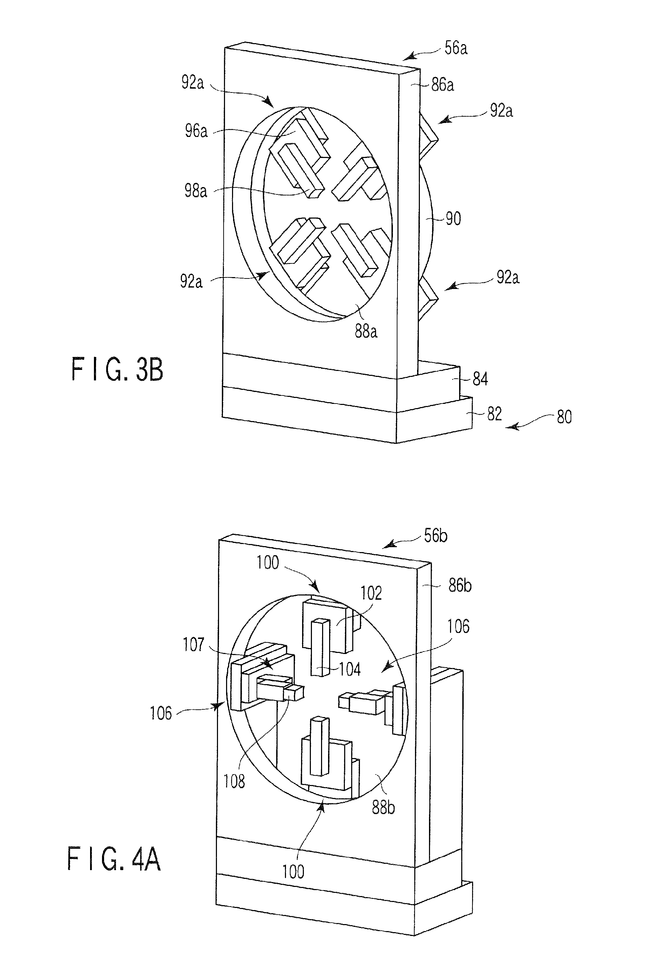

A manufacturing apparatus used for the method of manufacturing the bending tube 34 according to the present embodiment will now be described with reference to FIGS. 3A to 5D. The manufacturing apparatus has a forming assembly line 51 in which processing and assembling of the bending tube 34 is integrally performed. The forming assembly line 51 includes a bending part processor 52 arranged upstream of the line and a bending part connector 54 arranged downstream thereof. The bending part processor 52 successively forms the bending parts 36 from a plate material 111 by progressive press processing. The bending part connector 54 sequentially connects the formed bending parts 36. The bending part connector 54 is composed of a first chuck unit 56a located upstream of the line and a second chuck unit 56b located downstream thereof.

Referring to FIGS. 3A (the upstream side) and 3B (the downstream side), the first chuck unit 56a includes a moving unit 80 which is disposed on, for example, a floor. A moving sliding mechanism 82 of the moving unit 80 supports a moving base 84 slidably in the longitudinal axial direction of the line 51. The moving sliding mechanism 82 is formed of an actuator and a guide such as a cylinder, a motor and a ball screw, a linear motor, a shaft motor and others. A first chuck base 86a stands on the moving base 84 such as to be orthogonal to the above-mentioned longitudinal axis. A first circular opening 88a is formed through the first chuck base 86a, whose central axis extends along the forging longitudinal axis. A rotary connecting unit 90 is arranged on the periphery of the first circular opening 88a. The rotary connecting unit 90 includes four holding reducing-diameter units 92a separated by 90° about the foregoing longitudinal axis. The rotary connecting unit 90 enables the holding reducing-diameter units 92a to rotate about the above longitudinal axis. A holding reducing-diameter sliding mechanism 96a, constituting the holding reducing-diameter unit 92a, supports a holding reducing-diameter chuck 98a at the end thereof such that the holding reducing-diameter chuck 98a is slidable in the radial direction of the foregoing longitudinal axis. These holding reducing-diameter chucks 98a receive and hold the bending part 36 from the bending part processor 52 and reduce the diameter of the bending part 36. Similar to the moving sliding mechanism 82, each holding reducing-diameter sliding mechanism 96a is formed of an actuator and a guide such as a cylinder, a motor and a ball screw, a linear motor, as a shaft motor and others. An inner end face of the holding reducing-diameter chuck 98a has a recess whose curvature is substantially equal to or less than that of the bending part 36 to be held and reduced in diameter.

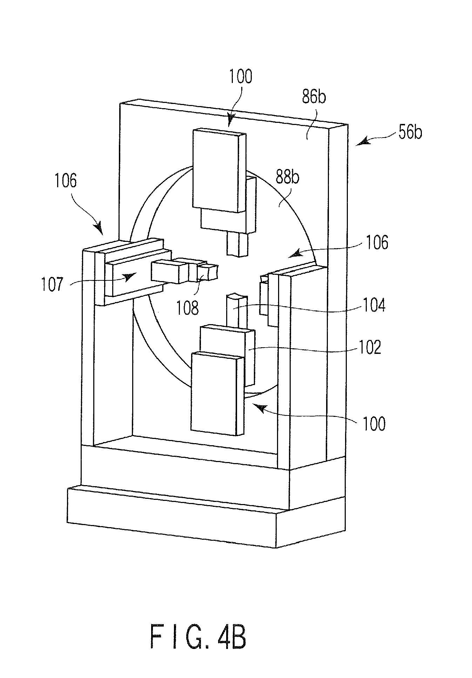

Referring to FIGS. 4A (the upstream side) and 4B (the downstream side), the second chuck unit 56b has a second chuck base 86b which stands orthogonal to the above-described longitudinal axis. A second circular opening 88b is formed through the second chuck base 86b, whose central axis extends along the foregoing longitudinal axis. On the periphery of the second circular opening 88b, a pair of holding units 100 is provided at symmetrical positions with respect to the foregoing longitudinal axis. A holding sliding mechanism 102, constituting the holding unit 100, supports a holding chuck 104 at the end thereof such that the holding chuck 104 is slidable in the radial direction of the foregoing longitudinal axis. These holding chucks 104 hold the uppermost-stream bending part 36 of a group of connected bending parts. Similar to the holding reducing-diameter chuck 98a, an inner end face of the holding chuck 104 has a recess whose curvature is substantially equal to or less than that of the held bending part 36. In addition, a pair of conveying units 106 is arranged on the periphery of the second circular opening 88b. The positions of the units 106 correspond to those obtained by rotating the pair of holding units 100 about the foregoing longitudinal axis by 90°. A conveying sliding mechanism 107, constituting the conveying unit 106, supports a conveying chuck 108 at the end thereof such that the conveying chuck 108 is slidable in both of the radial direction of the foregoing longitudinal axis and the longitudinal axial direction. These conveying chucks 108 hold the newly connected bending part 36 and convey it in the downstream direction together with the bending part group. Similar to the holding reducing-diameter chuck 98a and the holding chuck 104, an inner end face of the conveying chuck 108 has a recess whose curvature is substantially equal to or less than that of the bending part 36 to be held and conveyed. The conveying chuck 108 slides in the radial direction of the above-described longitudinal axis such that the conveying chuck 108 is not brought into contact with the associated holding reducing-diameter chuck 98a holding the bending part 36 received from the bending part processor 52.

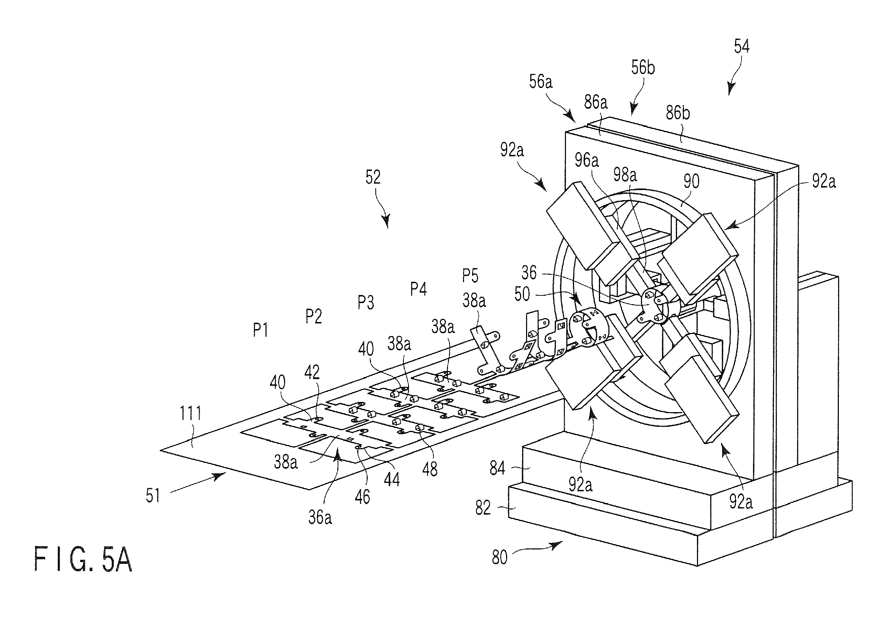

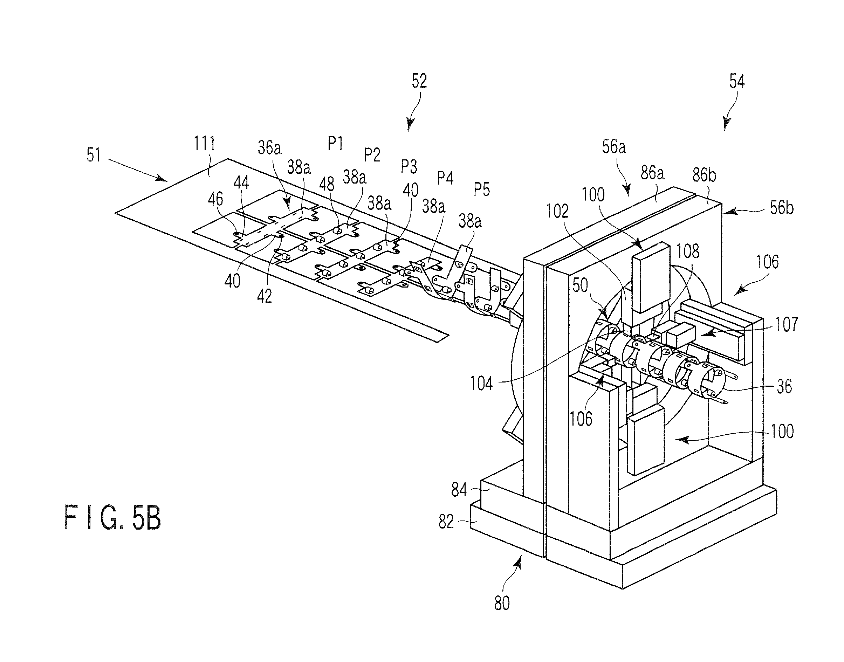

Referring to FIGS. 5A and 5B, the first and second chuck units 56a and 56b are arranged on the upstream and downstream sides of the forming assembly line 51.

The method of manufacturing the bending tube 34 will now be described in detail with reference to FIGS. 5A to 6H. Progressive press processing of the bending part 36 through the bending part processor 52 on the plate material 111 supplied from upstream will be described with reference to FIGS. 5A to 5D.

### Step 1 (First Processing Position P1)

In the elastic plate material 111, such as a metal plate material, a bending portion preparation portion 36a for forming the bending part 36 is formed by subjecting the portion around the bending portion preparation portion 36a to punching processing.

The bending portion preparation portion 36a includes a plate-shaped cylindrical portion preparation portion 38a, serving as a tubular portion preparation portion, for forming the cylindrical portion 38 of the bending part 36, which extends in the width direction of the plate material 111. The pair of projecting portion tongue piece portions 40 each having the projecting portion 42 extend from the front end of the cylindrical portion preparation portion 38a and the pair of receiving portion tongue piece portions 44 each having the through hole 46 extend from the back end thereof. Each tongue piece portion extends in the longitudinal direction of the plate material 111. One of the pair of receiving portion tongue piece portions 44 is located at substantially the middle point between the pair of projecting portion tongue piece portions 40 with respect to the width direction of the plate material 111. In the cylindrical portion preparation portion 38a, pairs of slits extend side by side in the width direction of the plate material 111 such that the positions of the pairs of the slits correspond to the upper, lower, left, and right position of the bending part 36. The pair of slits defines the front and back end faces of the wire holder 48.

### Step 2 (Second Processing Position P2)

A portion between the pair of slits is protruded inwardly from a side serving as an outer peripheral surface of the bending part 36 to a side serving as an inner peripheral surface thereof (from a lower side of the forming assembly line 51 to a upper side thereof) by bending processing, thus forming the wire holder 48.

### Step 3 (Third Processing Position P3)

In connecting portion between the cylindrical portion preparation portion 38a and the projecting portion tongue piece portion 40, Z-shape bending processing is carried out so that the projecting portion tongue piece portion 40 is arranged substantially parallel to the cylindrical portion preparation portion 38a on the side serving as an inner peripheral surface of the bending part 36 of the cylindrical portion preparation portion 38a (on the upper side of the forming assembly line 51). The height of the step provided by the Z-shape bending processing is slightly greater than the thickness of the plate material 111.

### Step 4 (Fourth Processing Position P4)

Portions connecting side portions of the plate material 111 with respect to the width direction to the cylindrical portion preparation portion 38a are removed by punching processing. In this instance, the central portion of plate material 111 with respect to the width direction and the cylindrical portion preparation portion 38a are remained connecting to each other.

### Step 5 (Fifth Processing Position P5)

The cylindrical portion preparation portion 38a of the bending part 36 is shaped into a cylinder by gradually bending from a side serving as an outer peripheral surface of the bending part 36 to a side serving as an inner peripheral surface thereof (from a lower side of the forming assembly line 51 to a upper side thereof), thus forming the cylindrical portion 38. After that, the portion connecting the central portion of the plate material 111 with respect to the width direction to the cylindrical portion preparation portion 38a are removed by punching proceeding, synchronously with holding of the cylindrical portion 38 through the holding reducing-diameter chucks 98a in step 6. The bending part 36 formed as described above has the discontinuous portion 50 which extends in the axial direction of the bending part 36 across the cylindrical portion 38 which extends in the peripheral direction.

### Step 6

The finished bending part 36 (hereinafter, simply referred to as a first bending part 36) which has been shaped into a cylinder in the fifth step and is send to the termination is transferred to the holding reducing-diameter chucks 98a of the first chuck unit 56a of the bending part connector 54.

A process of connecting the bending parts 36 in the bending part connector 54 will now be described.

### Step 6

The moving sliding mechanism 82 of the first chuck unit 56a moves the moving base 84 toward the upstream side of the forming assembly line 51, so that the first bending part 36 located at the termination of the bending part processor 52 is disposed at the middle of the first circular opening 88a of the first chuck base 86a. The holding reducing-diameter sliding mechanisms 96a slide the respective holding reducing-diameter chucks 98a inwardly in the radial direction of the foregoing longitudinal axis, thus holding the cylindrical portion 38 of the first bending part 36 through the holding reducing-diameter chucks 98a. Thus, the first bending part 36 after removing the portion connecting the central portion of the plate material 111 with respect to the width direction to the cylindrical portion 38 by punching processing is moved by moving the moving base 84 toward the downstream side of the forming assembly line 51 through the moving sliding mechanism 82.

### Step 7

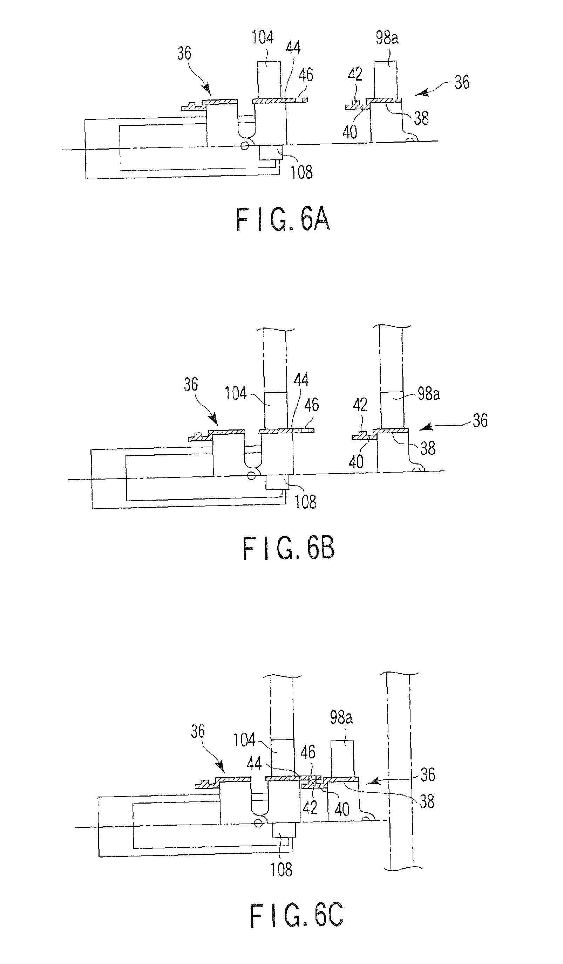

Referring to FIG. 6A, the holding chucks 104 of the second chuck unit 56b hold the cylindrical portion 38 of the uppermost-stream bending part 36 (hereinafter, simply referred to as a second bending part 36) of the bending part group. In this instance, the first and second bending parts 36 are held concentrically, the foregoing longitudinal axis serving as the central axis, and apart from each other.

In step 7, the rotation of the rotary connecting unit 90 causes the holding reducing-diameter chucks 98a to rotate about the foregoing longitudinal axis, so that the projecting portion tongue piece portions 40 of the first bending part 36 are aligned with the receiving portion tongue piece portions 44 of the second bending part 36 with respect to the peripheral direction of the foregoing longitudinal axis.

### Step 8

Referring to FIG. 6B, each holding reducing-diameter sliding mechanisms 96a further slides the associated holding reducing-diameter chuck 98a inwardly in the radial direction of the foregoing longitudinal axis and the holding reducing-diameter chuck 98a urge and thus elastically deform the first bending part 36. Consequently, the diameter of the cylindrical portion 38 of the first bending part 36 is reduced. At that time, the width of the discontinuous portion 50 of the first bending part 36 is reduced. If the amount of reduction in diameter of the cylindrical portion 38 of the first bending part 36 is large, the ends with respect to the circumferential direction of the cylindrical portion 38 of the first bending part 36 are superposed on each other.

### Step 9

Referring to FIG. 6C, the moving sliding mechanism 82 moves the moving base 84 toward the downstream side of the forming assembly line 51, so that the projecting portions 42 on the projecting portion tongue piece portions 40 of the first bending part 36 are aligned with the through holes 46 in the receiving portion tongue piece portions 44 of the second bending part 36 in the radial direction of the foregoing longitudinal axis.

### Step 10

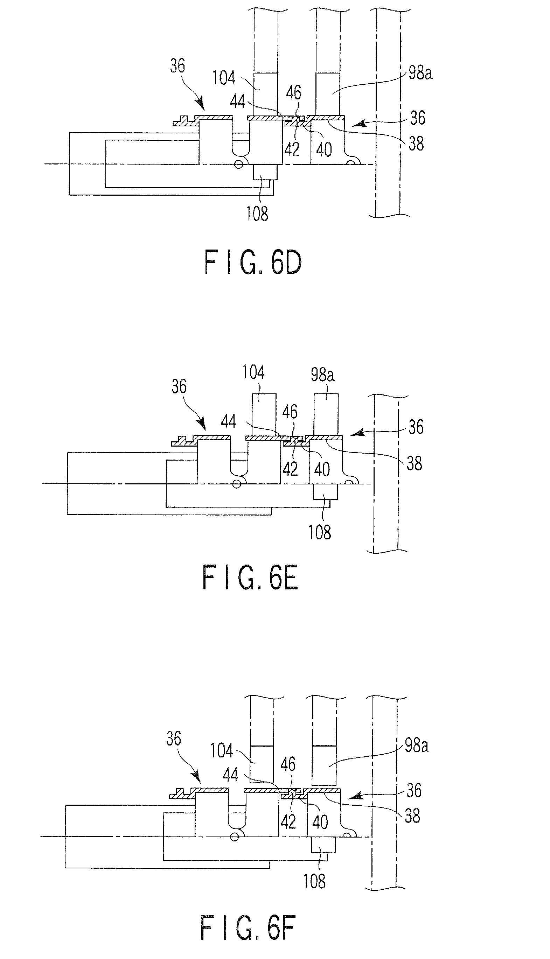

Referring to FIG. 6D, each holding-reducing sliding mechanism 96a slides the associated holding reducing-diameter chuck 98a outwardly in the radial direction of the foregoing longitudinal axis to release the urging of the first bending part 36 through the holding reducing-diameter chuck 98a and then the elastic deformation of the first bending part 36, thus returning the reduced diameter of the cylindrical portion 38 of the first bending part 36 to its original state. Consequently, the projecting portions 42 on the projecting portion tongue piece portions 40 of the first bending part 36 are moved outwardly in the radial direction of the foregoing longitudinal axis and are then inserted into the through holes 46 in the receiving portion tongue piece portions 44 of the second bending part 36.

### Step 11

Referring to FIG. 6E, each conveying sliding mechanism 107 moves the associated conveying chuck 108 toward the upstream side and aligns the conveying chuck 108 with the first bending part 36 with respect to the foregoing longitudinal axial direction. Further, each conveying sliding mechanism 107 slides the associated conveying chuck 108 inwardly in the radial direction of the foregoing longitudinal axis, so that the first bending part 36 is held by the conveying chucks 108.

### Step 12

Referring to FIG. 6F, each holding reducing-diameter sliding mechanism 96a slides the associated holding reducing-diameter chuck 98a outwardly in the radial direction of the foregoing longitudinal axis, thus releasing the first bending part 36. Similarly, each holding sliding mechanism 102 slides the associated holding chuck 104 outwardly in the radial direction of the above-described longitudinal axis, thus releasing the second bending part 36.

### Step 13

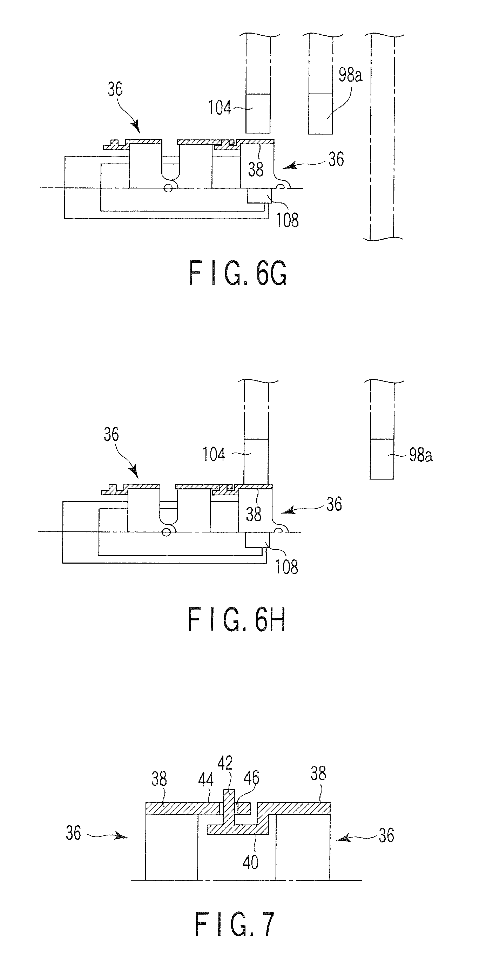

Referring to FIG. 6G, each conveying sliding mechanism 107 moves the associated conveying chuck 108 toward the downstream side, thus aligning the first bending part 36 with the holding chucks 104 with respect to the longitudinal axial direction.

### Step 14

Referring to FIG. 6H, each holding sliding mechanism 102 slides the associated holding chuck 104 inwardly in the radial direction of the foregoing longitudinal axis, thus holding the first bending part 36 through the holding chucks 104. Simultaneously, the moving sliding mechanism 82 moves the moving base 84 toward the upstream side of the forming assembly line 51, thus returning each holding reducing-diameter chuck 98a to the initial position.

### Step 15

Providing that the first bending part 36 newly connected to the bending part group is served as the second bending part 36, the above-described steps 1 to 14 are repeated.

The method of manufacturing the insertion portion 24 of the endoscope according to the present embodiment, therefore, has the following advantages.

In the manufacturing method according to the present embodiment, the first bending part 36 having the first cylindrical portion 38 which is elastic and substantially cylindrically shaped and the discontinuous portion 50 which extends across the first cylindrical portion 38 which extends in the peripheral direction is prepared on one side and the second bending part 36 having the second cylindrical portion 38 is prepared on the other side. The first cylindrical portion 38 is elastically deformed to change the diameter thereof. Further, the first and second bending parts 36 are moved relative to each other, so that the projecting portions 42, which are arranged in one of the first and second bending parts 36 and extend in the radial direction of the cylindrical portion 38, are aligned with the holes 46 in the other of the first and second bending parts 36. Further, the elastic deformation of the first cylindrical portion 38 is released to return the diameter thereof to its original state and the projecting portions 42 are rotatably inserted into the respective holes 46, thus connecting the first and second bending parts 36 such that they are swingable relative to each other. As described above, the method of manufacturing the bending tube 34 is simplified. Advantageously, the endoscope insertion portion 24 can be easily manufactured.

In the manufacturing method according to the present embodiment, the first bending part 36, connected to the second bending part 36, serves as the second bending part 36 in the next step. Thus, the bending tube 34 can be continuously formed. This leads to an increase in manufacturing efficiency of the endoscope insertion portion 24.

Further, to form the first bending part 36, the projecting portion 42 and the hole 46 are formed by press processing to the plate material 111, the first bending part preparation portion 36a which includes the projecting portion 42, the hole 46, the substantially plate-shaped cylindrical portion preparation portion 38a for forming the first cylindrical portion 38 is formed. In addition, the cylindrical portion preparation portion 38a is bent to form the first cylindrical portion 38 and the discontinuous portion 50. Accordingly, the bending parts 36 of the bending tube 34 can be processed and connected in the integrated forming assembly line 51, thus reducing the number of steps needed to produce the bending tube 34.

As for the connected bending parts 36, the discontinuous portion 50 thereof may be joined. As for a method of joining, spot welding using a laser beam, bonding, or welding may be used depending on a material for the bending part 36. Joining the discontinuous portion 50 leads to a continuous form of the cylindrical portion 38 of the bending part 36, thus increasing the stiffness of the bending part 36. The same may apply to the following embodiments.

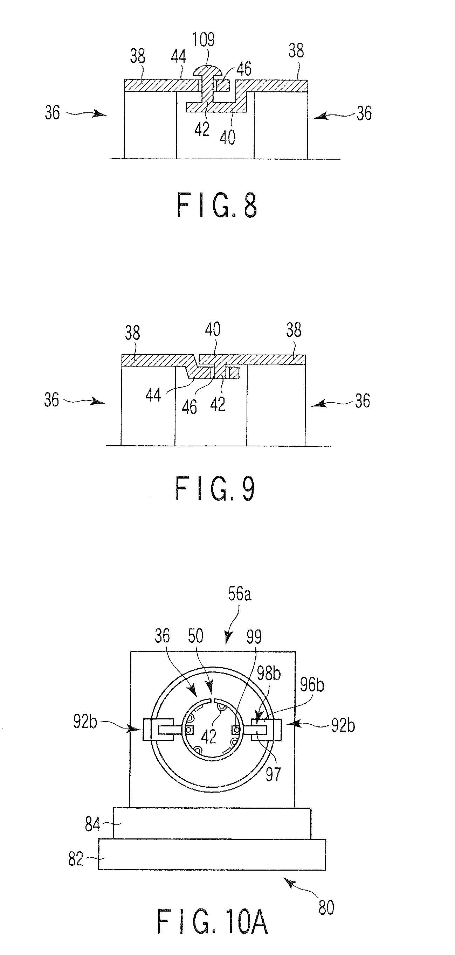

FIGS. 7 and 8 illustrate a second embodiment of the present invention. Components having the same functions as those of the first embodiment are designated by the same reference numbers and a description thereof is omitted.

Referring to FIG. 7, according to a method of manufacturing an insertion portion 24 of an endoscope in accordance with the present embodiment, when the projecting portion 42 of the first bending part 36 is inserted into the respective through hole 46 of the second bending part 36, the projecting portion 42 protrudes beyond the associated through hole 46. After the bending parts 36 are connected, as shown in FIG. 8, the protruding part of the projecting portion 42 is caulked or subjected to laser beam machining, thus forming a large diameter part 109 for preventing from falling off. According to the present embodiment, since the large diameter part 109 for preventing from falling off is arranged at the protruding part of the projecting portion 42, the bending parts 36 are strongly connected to each other.

FIGS. 9 to 10D illustrate a third embodiment of the present invention. Components having the same functions as those of the first embodiment are designated by the same reference numbers and a description thereof is omitted.

Referring to FIG. 9, in an endoscope 22 according to the present embodiment, the projecting portion tongue piece portion 40 is disposed substantially parallel to the cylindrical portion 38 of the first bending part 36. The projecting portion 42 projects from the inner surface of the projecting portion tongue piece portion 40 inwardly in the radial direction. In the connecting part between the cylindrical portion 38 and the receiving portion tongue piece portion 44 in the second bending part 36, the step projects inwardly in the radial direction such that the height of the step is greater than the thickness of the bending part 36. The outer surface of the receiving portion tongue piece portion 44 is arranged substantially parallel to the cylindrical portion 38 inside the inner surface of the cylindrical portion 38 in the radial direction.

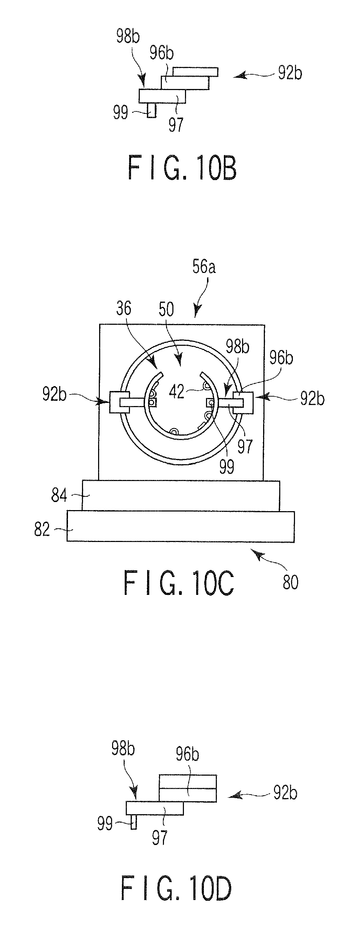

Referring to FIGS. 10A and 10B, in the first chuck unit 56a according to the present embodiment, holding increasing-diameter units 92b are arranged instead of the holding reducing-diameter units 92a in the first embodiment. Each holding increasing-diameter unit 92b has a holding increasing sliding mechanism 96b that enables a holding increasing chuck 98b to slide in the radial direction of the foregoing longitudinal axis. Each holding increasing chuck 98b has a block 97 extending inwardly in the radial direction of the foregoing longitudinal axis and a pin 99 extending in the longitudinal axial direction from the inner end portion of the block 97.

Referring to FIGS. 10A and 10B, to hold the first bending part 36 located at the termination of the bending part processor 52 through the holding increasing chucks 98b, the moving sliding mechanism 82 moves the moving base 84 toward the upstream side of the forming assembly line 51 and the pin 99 of each holding increasing chuck 98b is inserted into the first bending part 36. Then, each holding increasing sliding mechanism 96b slides the associated holding increasing chuck 98b outwardly in the radial direction of the foregoing longitudinal axis, thus holding the first bending part 36 through the pins 99 of the respective holding increasing chucks 98b.

Referring to FIGS. 10C and 10D, to connect the first bending part 36 to the second bending part 36, each holding increasing sliding mechanism 96b further slides the associated holding increasing chuck 98b outwardly in the radial direction of the foregoing longitudinal axis and the holding increasing chucks 98b urge and deform elastically the first bending part 36. This leads to an increase in diameter of the first bending part 36. At that time, the width of a discontinuous portion 50 of the first bending part 36 increases. After the projecting portions 42 of the first bending part 36 are aligned with the through holes 46 of the second bending part 36, each holding increasing sliding mechanism 96b slides the associated holding increasing chuck 98b inwardly in the radial direction of the foregoing longitudinal axis to release the urging through the holding increasing chucks 98b and then the elastic deformation of the first bending part 36, thus returning the diameter of the first bending part 36 to its original state. Consequently, each projecting portion 42 of the first bending part 36 is moved inwardly in the radial direction of the longitudinal axis and is then inserted into the corresponding through hole 46 of the second bending part 36.

The method of manufacturing the insertion portion 24 of the endoscope according to the present embodiment, therefore, has the same advantages as those of the first embodiment.

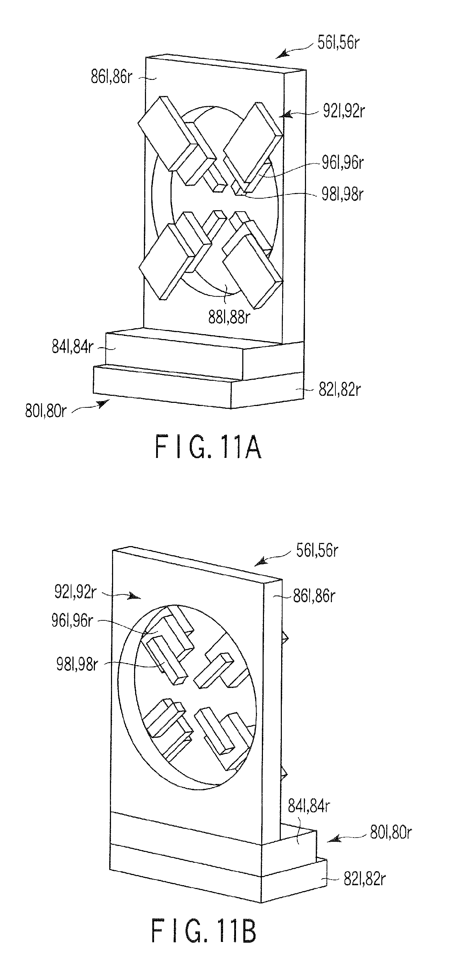

FIGS. 11A to 13D illustrate a fourth embodiment of the present invention. Components having the same functions as those of the first embodiment are designated by the same reference numbers and a description thereof is omitted.

A method of manufacturing a bending tube 34 of an insertion portion 24 of an endoscope according to the present embodiment will now be described.

A manufacturing apparatus used for the method of manufacturing the bending tube 34 in accordance with the present embodiment will be described with reference to FIGS. 11A to 13D. In the bending part processor 52 of the forming assembly line 51 of the manufacturing apparatus, a left line 51l and a right line 51r are arranged parallel to each other so as to have a mirror image relationship therebetween. A first left bending part 361 and a first right bending part 36r are formed through the left and right lines 51l and 51r, respectively. In the bending part connector 54 of the forming assembly line 51, the first left bending part 361 in the left line 511 and the first right bending part 36r in the right line 51r are alternatively connected to the uppermost stream bending part 36 of a group of connected bending parts.

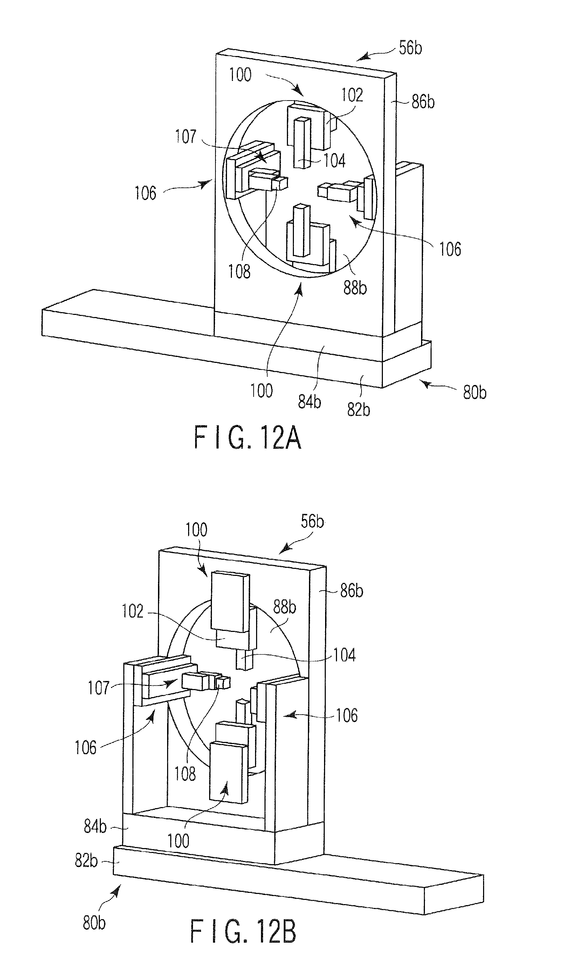

The bending part connector 54 includes a first left chuck unit 561, a first right chuck unit 56r, and a second chuck unit 56b, the first right and left chuck units 561 and 56r being located upstream of the assembly line and the second chuck unit 56b being disposed downstream thereof. Referring to FIGS. 11A (upstream side) and FIG. 11B (downstream side), each of the first left and right chuck units 561 and 56r is obtained by removing the rotary connecting unit 90 from the first chuck unit 56a in the first embodiment. In the first left and right chuck units 561 and 56r, components corresponding to those indicated at reference numbers Xa in the first chuck unit 56a are designated by reference numbers Xl and Xr and a description thereof is omitted. Referring to FIGS. 12A (upstream side) and 12B (downstream side), the second chuck unit 56b is obtained by adding a second moving unit 80b to the second chuck unit 56b in the first embodiment, the second moving unit 80b moving a second chuck base 86b in the width direction of the longitudinal axis of the forming assembly line 51. The second moving unit 80b includes a second moving sliding mechanism 82b and a second moving base 84b similar to the first moving unit 80 in the first embodiment.

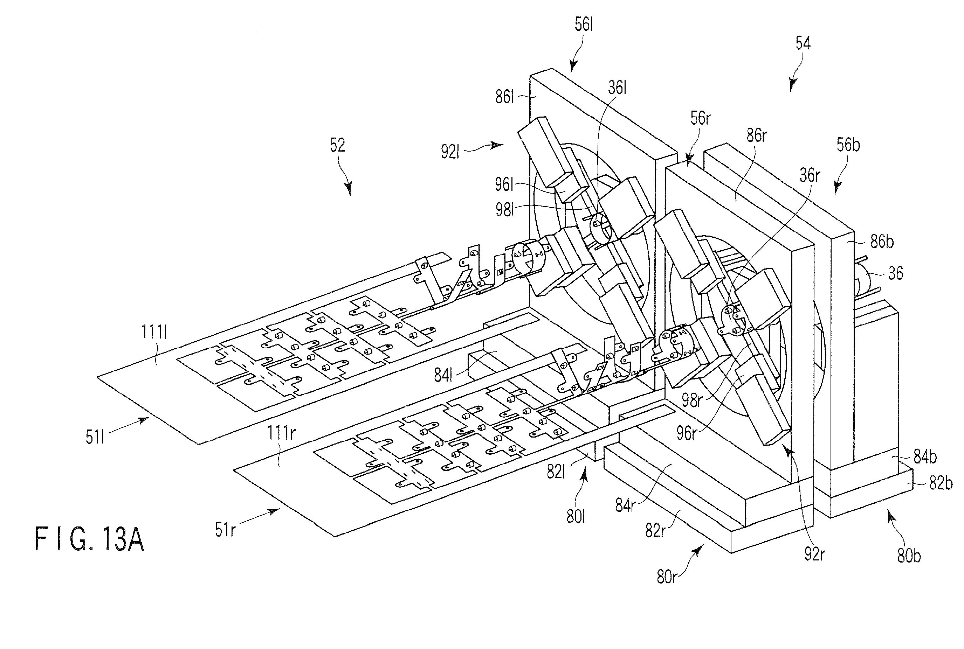

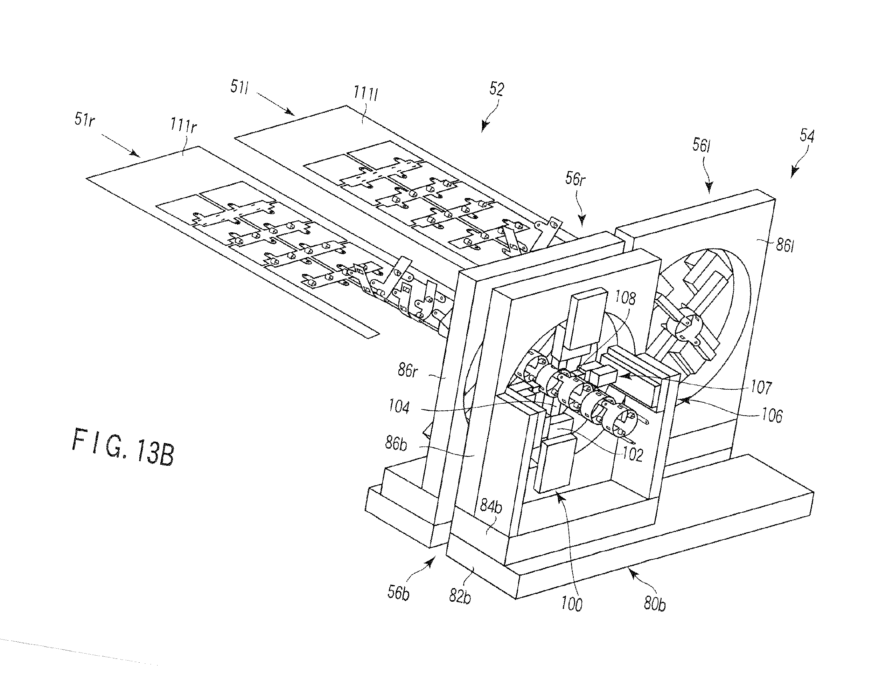

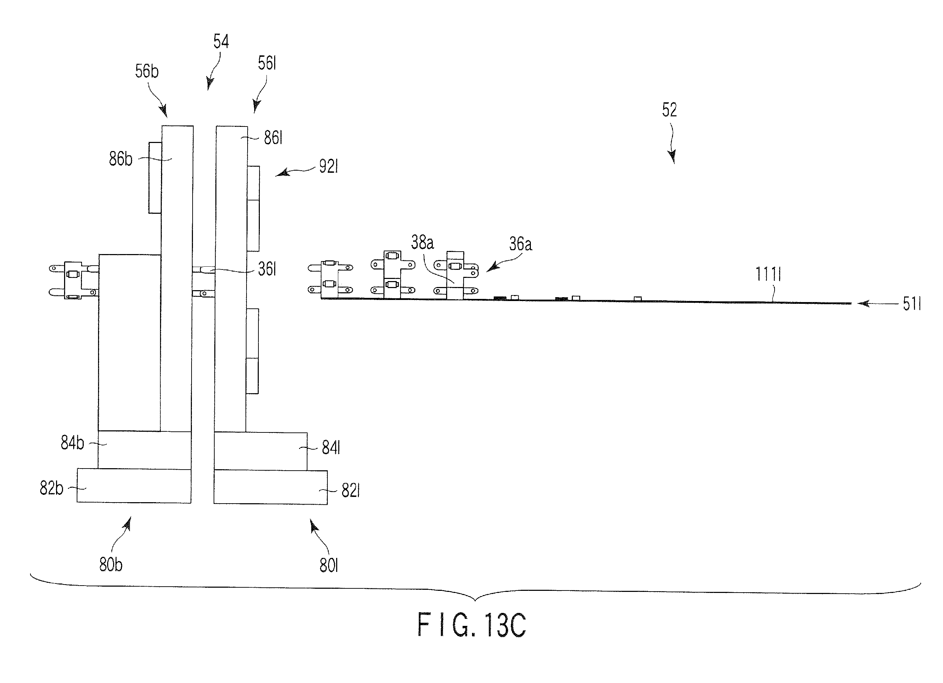

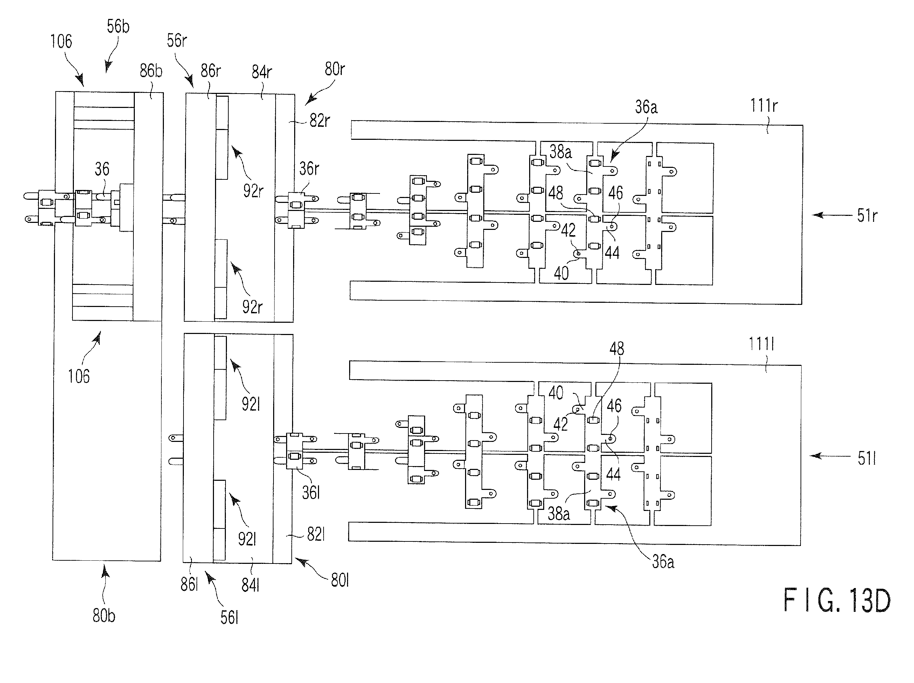

Referring to FIGS. 13A and 13B, the first left and right chuck units 561 and 56r are arranged side by side in the width direction of the above-mentioned longitudinal axis such that the first left and right chuck units 561 and 56r face the terminations of the left and right lines 51l and 51r of the bending part processor 52, respectively. The second chuck unit 56b is disposed downstream of the first left and right chuck units 561 and 56r. The second chuck base 86b of the second chuck unit 56b is moved between positions. A second circular opening 88b of the second chuck unit 56b faces a first left circular opening 881 of the first left chuck unit 561 in one position and a first right circular opening 88r of the first right chuck unit 56r in the other position.

A method of manufacturing the bending tube 34 will now be described with reference to FIGS. 13A to 13D. In the left and right lines 511 and 51r of the bending part processor 52, a first left bending part 361 and a first right bending part 36r which have a mirror-image relationship therebetween are formed from left and right plate materials 1111 and 111r, respectively, by progressive press processing in a manner similar to the first embodiment.

When a bending part 36 held through holding chucks 104 of the second chuck unit 56b is the second right bending part 36r, the moving sliding mechanism 82b moves the second moving base 84b, so that the second chuck base 86b is moved to a first left chuck base 861. The first left bending part 361 is connected to the second right bending part 36r through the first left chuck unit 561 and the second chuck unit 56b in a manner similar to the first embodiment. After that, the second chuck base 86b is moved to a first right chuck base 86r. The first right bending part 36r is connected to the second left bending part 361 through the first right chuck unit 56r and the second chuck unit 56b.

The method of manufacturing the insertion portion 24 of the endoscope according to the present embodiment, therefore, has the following advantages.

The manufacturing method according to the present embodiment uses steps of preparing first bending parts in the first and second lines, moving a second bending part between the first and second lines, aligning the first bending part in the first or second line with the second bending part, and connecting the first and second bending parts. These steps are suitable for processing and connecting the left and right bending parts 361 and 36r which have a mirror image relationship therebetween in the present embodiment.

A first modification of the fourth embodiment of the present invention will now be described below.

According to the present modification, the first chuck unit 56a is used instead of the first left and right chuck units 561 and 56r. This first chuck unit 56a is obtained by adding a first width moving unit to the first chuck unit 56a in the first embodiment. The first width-direction moving unit is used for moving the first chuck base 86a in the width direction of the longitudinal axis of the forming assembly line 51.

When a bending part 36 held through the holding chucks 104 of the second chuck unit 56b is the second right bending part 36r, the first and second chuck bases 86a and 86b are moved to the left line 51l of the bending part processor 52 by the first width-direction moving unit and the second moving unit 80b. The first left bending part 361 is connected to the second right bending part 36r by the first and second chuck units 56a and 56b. After that, the first and second chuck bases 86a and 86b are moved to the right line 51r and the first right bending part 36r is connected to the second left bending part 361 through the first and second chuck units 56a and 56b.

A second modification of the fourth embodiment of the present invention will now be described below.

In the fourth embodiment, the left and right lines 511 and 51r are arranged side by side substantially parallel to each other and the left and right chuck units 561 and 56r are also disposed side by side substantially parallel to each other. According to the present modification, the left and right lines 51l and 51r and the left and right chuck units 561 and 56r are radially arranged around the second chuck unit 56b. The moving sliding mechanism 82b rotates the second moving base 84b, so that the second chuck base 86b is rotated about its vertical axis between positions. The second chuck base 86b faces the first left chuck base 861 in one position and the first right chuck base 86r in the other position.

A fifth embodiment of the present invention will now be described below.

In the present embodiment, an insertion tube for forming an insertion tube portion 30 has the same structure as that of the bending tube 34 in the first embodiment. However, the insertion tube has no wire holders 48.

In a method for manufacturing an insertion portion 24 of an endoscope according to the present embodiment, the same manufacturing apparatus as that in the fourth embodiment is used. Each of first left and right chuck units 561 and 56r has the rotary connecting unit 90 similar to the first chuck unit 56a in the first embodiment. In the manufacturing method according to the present embodiment, in the bending part processor 52, bending parts 36 each having the same wire holders 48 as those in the first embodiment are formed in the left line 511 and bending parts 36 each having no wire holders 48 are formed in the right line 51r. The bending parts 36 each having the wire holders 48 are sequentially connected through the first left chuck unit 561 and the second chuck unit 56b in the bending part connector 54, thus forming a bending tube 34. Subsequently, the bending part 36 having no wire holders 48 is connected to the uppermost-stream bending part 36 of the bending tube 34 through the first right chuck unit 56r and the second chuck unit 56b and the subsequent bending parts 36 are sequentially connected, thus forming the insertion tube.

According to the present embodiment, the bending tube 34 and the insertion tube can be manufactured in the integrated forming assembly line 51. A process of manufacturing the insertion portion 24 of the endoscope is simplified. Furthermore, the number of parts of the endoscope insertion portion 24 is reduced.

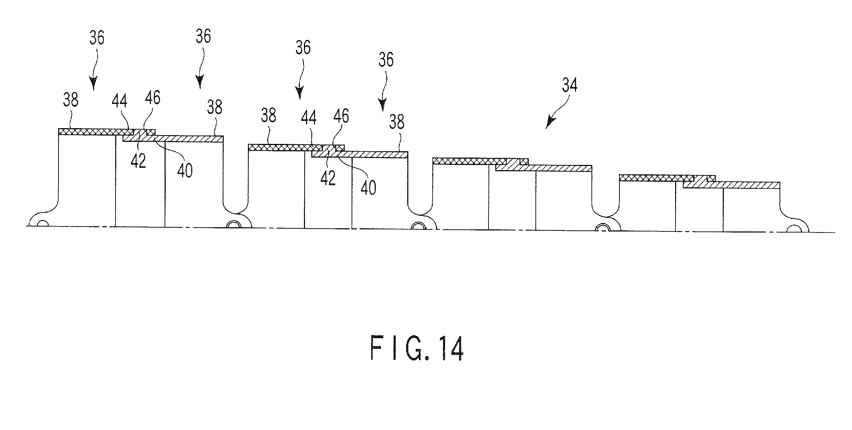

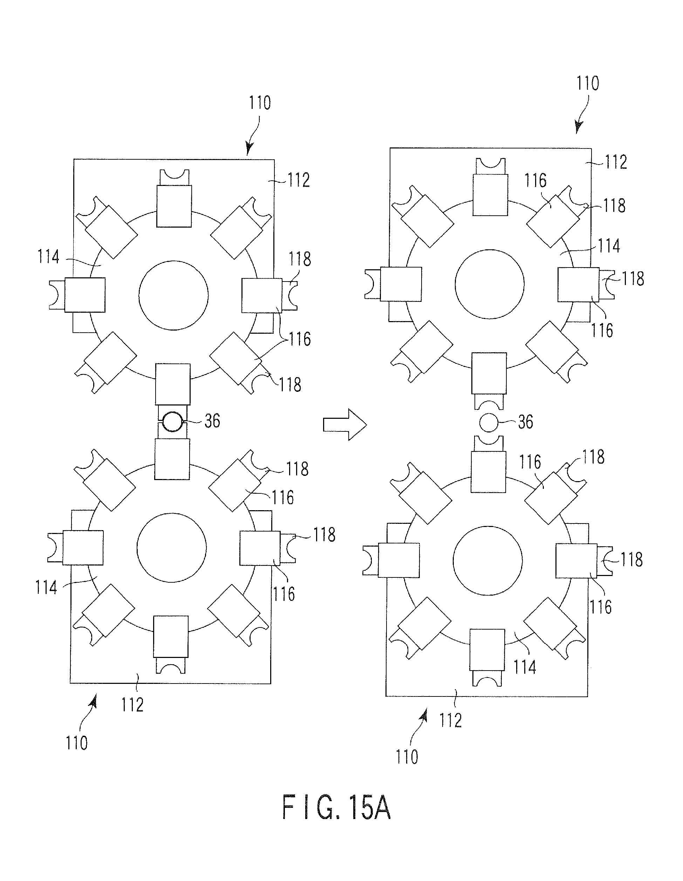

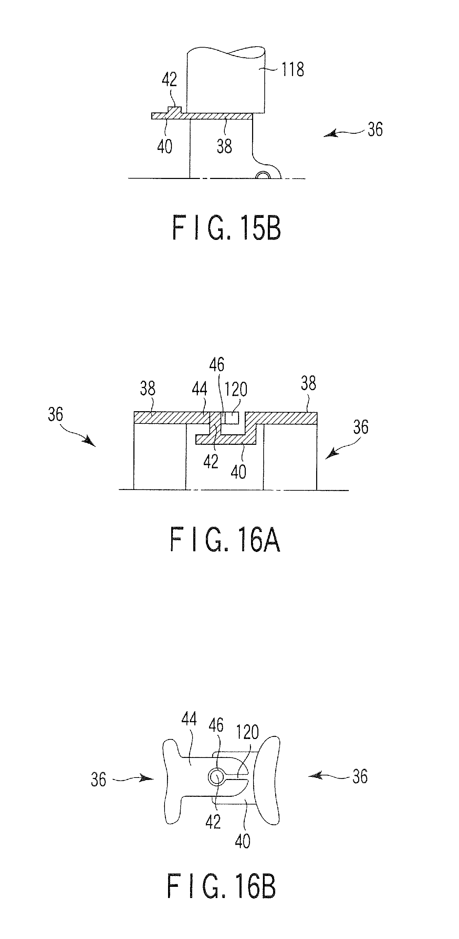

FIGS. 14 and 15 illustrate a sixth embodiment of the present invention. Components having the same functions as those of the first embodiment are designated by the same reference numbers and a description thereof is omitted.

Referring to FIG. 14, according to the present embodiment, the diameter of each bending part 36 is changed, thus varying the flexibility of a bending tube 34.

Referring to FIGS. 15, an apparatus for manufacturing the bending tube 34 of an insertion portion 24 of an endoscope according to the present embodiment includes a pair of press processing units 110 in the bending part processor 52. At the final stage of bending a cylindrical portion preparation portion 38a of a bending part 36, the press processing units 110 shape the cylindrical portion preparation portion 38a into a cylinder which has a predetermined diameter. The press processing units 110 are vertically movably disposed above and below the longitudinal axis of a forming assembly line 51. A rotating unit 112 of each press processing unit 110 has a disc-shaped rotating base 114 whose central axis is parallel to the above-mentioned longitudinal axis such that the rotating base 114 is rotatable about its central axis. Each rotating base 114 has a plurality of support blocks 116 on its periphery such that the support blocks 116 are apart from each other at regular intervals in the peripheral direction and project radially outwardly. Each support block 116 has a concave press die 118 at its projecting end portion. The respective press dies 118 define the outer surfaces of cylinders with different diameters.

In the method of manufacturing the bending tube 34 of the insertion portion 24 of the endoscope according to the present embodiment, the upper and lower press processing units 110 are used at the final stage of bending the cylindrical portion preparation portion 38a of the bending part 36. In other words, the upper and lower press dies 118 each having a desired diameter are selected and the rotating bases 114 of the upper and lower press processing units 110 are rotated so that the selected upper and lower press dies 118 face each other, with the cylindrical portion preparation portion 38a of the bending part 36 therebetween. The upper press die 118 is moved downward and the lower press die 118 is moved upward, thus press the cylindrical portion preparation portion 38a of the bending part 36 through the upper and lower press dies 118. At that time, as shown in FIG. 15B, each projecting portion 42 is prevented from being pressed through the press dies 118. When the diameter of each press die 118 is large, the diameter of the bending part 36 becomes large, so that the width of a discontinuous portion 50 is relatively large. On the other hand, when the diameter of each press die 118 is small, the diameter of the bending part 36 becomes small, so that the width of the discontinuous portion 50 is relatively small or ends of the cylindrical portion 38 with respect to the peripheral direction are overlapped.

The method of manufacturing the insertion portion 24 of the endoscope according to the present embodiment has the following advantages.

In the manufacturing method according to the present embodiment, the first bending part preparation portion 36a is bent by press processing, thus forming the first cylindrical portion 38 having an arbitrary diameter. Therefore, changing the diameters of the bending parts 36 can vary the flexibility of the bending tube 34.

In the above-described embodiments, the through hole 46 is used as a receiving portion 46. A recess 46 to which the projecting portion 42 is rotatably inserted may be used. Each bending part 36 which has the projecting portions 42 is reduced or increased in diameter and is then connected to another bending part 36. Each bending part 36 which has the through holes 46 may be reduced or increased in diameter and then be connected to another bending part 36. The bending parts 36 of one kind are connected. Two kinds of bending parts 36 may be connected. For example, a bending part having receiving portion tongue piece portions 44 extending from both end faces thereof and a bending part having projecting portion tongue piece portions 40 extending from both end faces thereof may be used. In this case, in a bending part forming section, different kinds of bending parts are formed through a plurality of lines and are connected in a manner similar to the fourth embodiment. Furthermore, another construction in which press processing can be performed by each support block 116 instead of the entire press processing unit 110 may be used.

FIGS. 16A and 16B illustrate a reference embodiment of the present invention. Components having the same functions as those in the first embodiment are designated by the same reference numbers and a description thereof is omitted.

In each bending part 36 according to the reference embodiment, each elastic receiving portion tongue piece portion 44 has a slit 120 which extends from the end of the receiving portion tongue piece portion 44 to a through hole 46 in the axial direction of the bending part 36. To connect the bending parts 36, both the bending parts 36 are moved closer to each other in the axial direction thereof, each projecting portion 42 of one of the bending parts 36 is inserted into the associated slit 120 in the receiving portion tongue piece portion 44 of the other bending part 36, and the projecting portion 42 is moved through the slit 120 to the through hole 46.

In the above-described embodiments, a metal plate material is pressed to form a bending part, but the material is not intended to be limiting in any way. A resin plate material may be used. Bending parts may be made of resin with the same structure and be arranged and then be connected by elastic deformation. Thus, the same advantages can be obtained. Furthermore, the through hole 46 serving as the receiving portion formed in the plate material 111 in the above-described embodiments may be formed by laser beam machining or etching. Moreover, the projecting portion 42 formed on the plate material 111 may be formed by etching the projecting portion tongue piece portion 40 in the vicinity of the projecting portion 42. In addition, the plate material 111 may be pre-processed by laser beam machining or etching so that the plate material 111 includes the bending part preparation portions 36a, and after that, the processed plate material 111 may be supplied to the forming assembly line 51.

## Claims

1. A method of manufacturing an insertion portion of an endoscope, **characterized by** comprising:
preparing a first bending part which includes a first tubular portion which is elastic and is substantially tube-shaped, and a discontinuous portion which extends across the first tubular portion which extends in a peripheral direction;
preparing a second bending part which includes a second tubular portion which is substantially tube-shaped;
changing a diameter of the first tubular portion by deforming the first tubular portion elastically;
aligning a projecting portion provided with one of the first and second bending parts and extending in a radial direction of the tubular portion with a receiving portion provided with the other of the first and second bending parts by moving the first and second bending parts relatively; and
connecting the first and second bending parts swingably relative to each other by releasing the elastic deformation of the first tubular portion to return the diameter thereof to its original state and inserting the projecting portion into the receiving portion rotatably.

2. The method of manufacturing an insertion portion of an endoscope according to claim 1,
**characterized in that**
the first bending part has the projecting portion which projects outwardly in a radial direction of the first tubular portion, and
changing the diameter of the first tubular portion includes reducing the diameter of the first tubular portion such that the projecting portion is moved inwardly in the radial direction of the first tubular portion.

3. The method of manufacturing an insertion portion of an endoscope according to claim 1,
**characterized in that**
the first bending part has the projecting portion which projects inwardly in a radial direction of the first tubular portion, and
changing the diameter of the first tubular portion includes increasing the diameter of the first tubular portion such that the projecting portion is moved outwardly in the radial direction of the first tubular portion.

4. The method of manufacturing an insertion portion of an endoscope according to claim 1,
**characterized in that**
the first bending part has the receiving portion,
the second bending part has the projecting portion which projects outwardly in a radial direction of the second tubular portion, and
changing the diameter of the first tubular portion includes increasing the diameter of the first tubular portion such that the receiving portion is moved outwardly in a radial direction of the first tubular portion.

5. The method of manufacturing an insertion portion of an endoscope according to claim 1,
**characterized in that**
the first bending part has the receiving portion,
the second bending part has the projecting portion which projects inwardly in a radial direction of the second tubular portion, and
changing the diameter of the first tubular portion includes reducing the diameter of the first tubular portion such that the receiving portion is moved inwardly in a radial direction of the first tubular portion.

6. The method of manufacturing an insertion portion of an endoscope according to claim 1, **characterized by** further comprising: repeating all of the steps, wherein the first bending part connected to the second bending part in the connecting serves as the second bending part in the next repeated steps.

7. The method of manufacturing an insertion portion of an endoscope according to claim 6, **characterized in that** the preparing the first bending part includes: forming the projecting portion and the receiving portion in a plate material, forming a first bending part preparation portion including the projecting portion, the receiving portion and a first tubular portion preparation portion for forming the first tubular portion, which is substantially plate-shaped; and forming the first tubular portion and the discontinuous portion by bending the first tubular portion preparation portion.

8. The method of manufacturing an insertion portion of an endoscope according to any one of claims 1 to 7, **characterized in that**
the preparing the first bending part includes preparing the first bending part in a first line and preparing the first bending part in a second line, and
the aligning and the connecting includes moving the second bending part between the first and second lines and positioning and connecting the second bending part to the first bending part in the first line or the first bending part in the second line.

9. The method of manufacturing an insertion portion of an endoscope according to claim 7,
**characterized in that**
the forming the first tubular portion includes forming the first tubular portion with an arbitrary diameter by bending the first tubular portion preparation portion through a press processing.

10. The method of manufacturing an insertion portion of an endoscope according to any one of claims 1 to 7, **characterized by** further comprising joining the discontinuous portion in the first bending part after the connecting the first and second bending parts.
